Europäisches Patentamt

European Patent Office

Office européen des brevets

(11) Publication number: **0 257 007 B1**

# EUROPEAN PATENT SPECIFICATION

(45) Date of publication of patent specification: **14.10.92**  (51) Int. Cl.5: **A61K 31/19, A61K 9/02**

(21) Application number: **87850240.0**

(22) Date of filing: **06.08.87**

The file contains technical information submitted after the application was filed and not included in this specification

(54) **Agent for treating conditions in the vagina.**

(30) Priority: **07.08.86 SE 8603338**

(43) Date of publication of application:
**24.02.88 Bulletin 88/08**

(45) Publication of the grant of the patent:
**14.10.92 Bulletin 92/42**

(84) Designated Contracting States:
**BE DE FR GB LU NL SE**

(56) References cited:
**DE-A- 3 303 018**
**DE-A- 3 510 531**
**GB-A- 253 918**
**GB-A- 272 543**
**GB-A- 2 112 285**

**CHEMICAL ABSTRACTS, vol. 105, 1986, Columbus, OH (US); p. 396, no. 49063y.**

(73) Proprietor: **Kabi Pharmacia AB**
**Rapsgatan 7**
**S-751 82 Uppsala(SE)**

(72) Inventor: **Kabi Pharmacia AB**
**Rapsgatan 7**
**S-751 82 Uppsala(SE)**

(74) Representative: **Hagelberg, Anders Torvald Sverker et al**
**Albihn West AB Stora Nygatan 15**
**S-411 08 Göteborg(SE)**

## Description

The present invention relates to an agent for reinforcing the natural protection mechanisms of the vagina mucosa by helping to re-establish the productive environment for lactobacteria, the agent being in the form of a cream, a gel or vaginal suppository and is containing lactic acid with buffering substance, with the content of lactic acid and buffering substance such that the pH lies within the range from 3.5 to 4, preferably close to 3.8, the agent containing a growth substrate for lactic acid bacteria and carrier vehicles which are of a type which is inert in this connection and which provide cream- or gel-like consistency or, alternatively, a substance which melts at body temperature and in body fluid.

It is known that an acid environment in the vagina of a woman is of crucial importance for protection against infection. This acid environment is maintained by a lactobacterial flora which produces lactic acid. In order for this production of lactic acid to be maintained to provide the pH reduction necessary for protection against infection, a favourable environment is needed for the bacterial flora.

A favourable environment of this type is generally present and results in a pH of approximately 4 in the mucous membrane secretions. However, the environment can be disturbed by factors such as variations in the oestrogen concentration during certain periods of the menstrual cycle and during the menopause and also by an increasing occurrence of secretions of various types, for example from protracted menstrual haemorrhages, premenstrual bloody discharges, mid-cycle bleeding and ejaculate. Foreign bodies in the vagina, for example coils, pessaries and those inserted, for example, in connection with antibiotic treatment and on washing can also lead to a disruption of the bacterial flora which is such that the pH rises. When the pH rises above 4.5 there is an increased tendency towards growth of anaerobic bacteria.

Disruptions of the bacterial flora in the vagina without inflammatory reactions in the mucous membrane are referred to as vaginosis (NSV) and lead to offensive discharges and pH's over 4.5, but they are not generally regarded as an actual disorder. However, vaginosis is an indication that the defence against infection, which defence depends on a low pH, has been weakened and, in unfavourable circumstances, this leads to actual infection.

It is known to use agents in an attempt to cure infections by seeking to re-establish an acid environment in the vagina by supplying lactic acid. In this connection the first aim has been to create an acid environment and to counterbalance the more alkaline environment by artificial supply of lactic acid. On the basis of this assumption, attempts were therefore made to use extraneous sources to replace the lactic acid no longer provided to a sufficient extent by the bacterial flora in the vagina. In this connection agents were used which destroyed the lactic acid production which was dependent on environment. Moreover, in many cases substances were supplied such as preservatives for the product and agents intended to inhibit growth of pathogenic bacteria in the vagina, which agents were of a type which further impaired the environment for lactobacteria. This created a condition in which the natural mechanism with symbiosis between bacterial flora and tissue was lost. This is particularly unfortunate when those products are used for treatment of vaginosis which cannot be regarded as an actual disorder in itself, but which can result in such a disorder or in a chronic abnormal condition when agents are supplied which were intended to act as medicaments but which make it difficult to re-establish the natural advantageous environment.

In GB-A-253 218 is described a process for production of a disinfecting agent including buffered lactic acid and indicated to be suitable for disinfecting, antiseptic, preserving or like purposes. It is also suggested that the agent is admixed with nutrient materials.

Improvement of this process for the production of the disinfecting agent is described in GB-A-272 543 in which publication it is suggested that the disinfecting agent is converted to semi-solid or solid form, such as by mixing the agents with gelatin with or without the addition of glycerin.

None of these publications are disclosing any specified composition containing lactic acid and nutrient material but only the presence of a nutrient is suggested.

In Chemical Abstracts, Vol. 105, 1986, Abstract No 49063y is disclosed a pharmaceutical composition specified to its content. The composition contains lactic acid and lactose as nutrient. The proportions between lactic acid and lactose is from 1.5:20.0 to 3:5.0. The composition contains also conservatives such as p-aminobenzoate and thymidine. As the form of use suppositories are mentioned.

In connection with the present application it has been shown, that for treating conditions as vaginosis and even vaginitis in the vaginal mucosa a cream or gel or a vaginal suppository containing buffered lactic acid could be very useful by re-establish a colonization of lactobacterial flora. In order to stabilize this flora to reach a long-lasting natural condition of the mucosa it has been shown that it is of great value to combine the lactic acid with a nutrient for the lactobacteria. Advantageous nutrient materials in this connection is glycogen because it is a preferred substrate for the bacteria fermentation.

However, the nutrient material presented in the vaginal environment will increase the risk for growth of microorganisms especially fungus as candida. Already by the storing of a pharmaceutical agent containing nutrient materials, as the composition according to the mentioned Chemical Abstract, a colonization of harmful bacteria can occur and of course this risk is increased when the proportion of nutrient materials is high. By the proportions suggested in said Chemical Abstract it would be necessary to add to the composition preservatives for the product, which, according to what have been discussed above, will disturb the colonization of lactobacteria. Such preservatives is also prescribed in the receipt disclosed in the publication.

The object of the present invention is to provide an agent for treating conditions in the vagina mucosa as vaginosis, which agent can be produced without any harmful disinfecting agents and without the risk to being a nutrient for harmful micro organisms.

The object of the invention is achieved by incorporating lactic acid in a considerably greater weight proportion than the growth substrate, for example in weight ratios of 20:1 down to 500:1 preferably 50:1.

The agent according to the invention has a cream or gel consistency or is given the form of a vaginal suppository. The agent contains as its main constituents:

Lactic acid supplemented (buffered) with sodium hydroxide to give a pH of 3-4. The pH is advantageously close to 4 and is not so low that the acid irritates the tissues or impairs the environment for the lactobacteria which require a moderately acid environment. A pH of 3.7-3.8 has been shown to be particularly advantageous.

Glykogen as the growth substrate for the lactobacteria, so that the treatment not only results in adjustment of the pH level to a lower figure by means of supplying lactic acid, but also to re-establishment of an advantageous environment for the growth of the lactobacteria in order to regenerate the natural conditions.

A vehicle for the active constituents of the agent has to be added to form a usable pharmaceutical product. One such suitable vehicle for a cream is propylene glycol, but other substances of an inert nature are also known to be of use in this connection as vehicles.

Consistency agent such as, in the case of creams and gels, could be methyl hydroxypropyl ether of cellulose. As an example of a commercial product there may be mentioned Hypromellosum® 90 HG 4000.

If a vaginal suppository is to be produced, a vehicle is required which makes it possible to form an article which is relatively solid at room temperature and in a dry environment, but which melts at body temperature and in contact with body fluid. In this connection there may be used polyethylene glycol, PEG, which gradually melts in the vaginal environment.

A more specific composition emerges from the following examples.

Agent according to the invention:

Example 1. Cream

| Lactic acid | 5.0 g |
| Na OH ad. pH 3.9 | |
| Glycogen | 0,1 g |
| Propylene glycol (85%, remainder $H_2O$) | 15.0 g |
| Methyl hydroxypropyl ether of cellulose e.g. Hypromellosum® 90 HG 4000 (2.5%, remainder $H_2O$) | |

Example 2. Cream

| Lactic acid | 5.0 g |
| Na OH (5M) | 4.1 g |
| Glycogen | 0.1 g |
| Propylene glycol (85%, remainder $H_2O$) | 15.0 g |
| Methyl hydroxypropyl ether of cellulose e.g. Hypromellosum® 90 HG 4000 (2.5%, remainder $H_2O$) | ad. 100. 0 g |

Comparison tests

3

The object was to investigate the effects of various cream compositions compared to the effect of the cream according to Example 1 of the invention on the growth of lactobacteria and also other bacteria generally present in the vagina, such as Staphylococcus aureus, Pseudomonas, Candida albicans, Clostridia, E.coli and Enterococci.

Agents, which are not the subject of the invention and are previously known, are given for comparison:

| a. | Lactic acid | 5.0 g |
| | Na OH, ad. pH 3.9 | |
| | Propylene glycol (85%, remainder $H_2O$) | 15.0 g |
| | Hypromellosum® 90 HG 4000 (2.5%, remainder $H_2O$) | ad. 100 g |
| b. | Lactic acid | 5.0 g |
| | Na OH, ad. pH 3.5 | |
| | Propylene glycol (85%, remainder $H_2O$) | 15.0 g |
| | Hypromellosum® 90 HG 4000 (2.5%, remainder $H_2O$) | ad. 100 g |
| c. | Conc. acetic acid | 920 mg |
| | Ricinoleic acid | 750 mg |
| | Sulph. oxyquinoline | 25 mg |
| | Glycerol, gum arabic | |
| | Potassium hydroxide | |
| | Egg albumen mix | |
| | Tragacanth | |
| | Preservative (propyl parahydroxybenz., stannous chloride) | |
| | Odorant | |
| | Aq. purif. q.s. - pH 4.0 | |

The following bacteria were tested

The initial bacteria count was $1 \times 10^9$. Staph. aureus, Pseudomonas aeruginosa, Candida albicans, Clostr. sporogenes, EF-15941, Lactobacillaceae var. rhamnosus, EG 18011, E.coli, Enterococcus-spec. 9739.

0.5 g of the various cream compositions were placed in sterile plastic tubes. The bacteria species were suspended in physiological saline solution at a rate of $1 - 10^9$/ml. 0.5 ml of the suspension was transferred to the various cream compositions and then thoroughly shaken in a mixer. It was then cultured directly onto agar plates. The plates were then placed in a thermostat at 37° for 1-2 days, after which a reading was taken. Culturing onto the plates was carried out directly in part and then after 30 minutes, 1 hour, 2 hours, 4 hours, 6 hours, 7 hours, 8 hours, 10 hours, 12 hours, 16 hours, and then each day.

Result

The results show that there was no great difference between lactic acid creams without glycogen at a pH of 3.5 compared with a pH level of 3.9 in respect of the time taken for all the bacteria tested in the study to be killed.

The content of preservative (in agent c) provided for a more rapid killing of lactobacteria than creams which did not contain any preservatives. The lactobacteria survived twice as long if the lactic acid cream contained 0.5 g glycogen than if it contained no glycogen.

E.coli survived considerably longer in agent c compared with the three lactic cid creams. Staph. aureus and Pseudomonas aeruginosa did not survive any longer than six hours in any of the creams. On the other hand, there was no effect on the growth of Candida albicans and Clostr. sporogenes.

This shows that it is of great importance for the balance between the microorganisms in the vagina to be maintained. Above all, it is important that the lactic acid bacteria are allowed to predominate in the vaginal content, since these bacteria confer a natural protection against infection by virtue of their ability to form lactic acid and thereby acidify the vaginal content.

In the study mentioned various cream compositions were tested in respect of the survival of the lactic acid bacteria in vitro in addition to other bacteria which are generally present but which have pathogenic properties. It emerged from the study that preservatives in the cream do not favour the growth of the lactobacteria. After only 1 hour all lactobacteria in agent c were killed. In the creams which contained only lactic acid, the lactobacteria survived considerably longer and, in addition, longer in creams with the lowest pH. On addition of 0.1 g glycogen, the lactobacteria survived twice as long compared with the creams which contained only lactic acid.

The situation as regards the effects of the various creams on the composition of the bacterial flora in vivo is of course different. The pH in a lactic acid cream should be set lower than the pH value for lactic acid (pH = 3.87) in order to better withstand the expected alkaline charge. In accordance with the abovementioned results, the pH of a cream for treating vaginosis should be set somewhat lower than 3.8 by the addition of glycogen. Glycogen is found in abundance in the vaginal epithelial cells in fertile women and is an important nutrient substrate for the lactobacteria. In addition, the cream should not contain any preservatives. The requirements for creams made without preservatives are of course that undesired bacteria do not grow in the cream tubes during storage. Storage tests on the abovementioned lactic acid creams showed that after 16 weeks of storage there was no fungal growth or bacterial growth.

In the treatment of vaginosis (offensive discharges caused by imbalance in the vaginal flora without inflammation) it is important to re-establish the lactobacterial flora. For this purpose the invention has provided an acidic lactic acid cream containing glycogen without preservatives. Compared with creams which contain only lactic acid (agents a and b) or acidic creams with preservatives (agent c) the cream according to the invention has been shown in vitro to have twice as favourable an effect on the survival of the lactobacteria. The agent according to the invention can thus be used for re-establishing a normal environment in cases of vaginosis, whereas previously known agents lead to a risk of the lactic acid production being lost. Intestinal bacteria and Staph. aureus have not survived for more than 4 and 6 hours respectively in the cream according to the invention.

## Claims

1. Agent for reinforcing the natural protection mechanisms of the vagina mucosa by helping to re-establish the productive environment for lactobacteria, the agent being in the form of a cream, a gel or vaginal suppository and containing lactic acid with buffering substance, with the content of lactic acid and buffering substance such that the pH lies within the range from 3.5 to 4, preferably close to 3.8, the agent containing a growth substrate for lactobacteria and carrier vehicles which are of a type which is inert in this connection and which provide a consistence adapted to the form of the agent, characterized in that the lactic acid has a weight proportion compared to the growth substrate, in the weight ratios of 20:1 down to 500:1 preferably 50:1, the growth substrate being glycogen.

2. Agent according to Claim 1, characterized in that its content is essentially as follows:

| | |
|---|---|
| Lactic acid | 5.0 g |
| Na OH for buffering the lactic acid to a pH of essentially 3.7-3.8 | |
| Glycogen | 0.1 g |
| Propylene glycol (85%, remainder $H_2O$) | 15.0 g |
| Consistency agent such as methyl hydroxypropyl ether of cellulose | ad.100 g |

giving a gelform.

3. Agent according to Claim 1, characterized in that its content is essentially as follows:

| | |
|---|---|
| Lactic acid | 5.0 g |
| Na OH (5M) | 4.1 g |
| Glycogen | 0.1 g |
| Propylene glycol (85%, remainder $H_2O$) | 15.0 g |
| Consistency agent such as methyl hydroxypropyl ether of cellulose (2.5%, remainder $H_2O$) | ad.100 g |

giving a cream form.

## Patentansprüche

1. Mittel zur Verstärkung des natürlichen Schutzmechanismusses der Vagina mucosa durch Unterstützung der Wiederbildung des produktiven Milieus für Milchsäurebakterien, wobei das Mittel in Form einer Creme, eines Gels oder vaginaler Zäpfchen vorliegt und Milchsäure mit einer Puffersubstanz enthält, wobei der Gehalt an Milchsäure und der Puffersubstsanz so ist, daß der pH-Wert im Bereich von 3,5 - 4, vorzugsweise nahe 3,8 liegt und daß das Mittel ein Wachstumssubstrat für Milchsäurebakterien und Träger enthält des Typs, der in dieser Verbindung inert ist und der eine Konsistenz bewirkt, die der Form des Mittels angepaßt ist,
dadurch gekennzeichnet,
daß die Milchsäure ein Gewichtsverhältnis bezogen auf das Wachstumssubstrat von 20 : 1 bis 500 : 1, vorzugsweise 50 : 1 hat und das Wachstumssubstrat glycogen ist.

2. Mittel nach Anspruch 1,
dadurch gekennzeichnet,
daß die Zusammensetzung im wesentlichen wie folgt ist:

| Milchsäure | 5,0 g |
| NaOH zum Puffern der Milchsäure auf einen pH-Wert von im wesentlichen | 3,7 - 3,8 |
| Glycogen | 0,1 g |
| Propylenglycol (85 % Rest H$_2$O) | 15,0 g |
| Konsistenzmittel wie Methylhydroxypropyläther aus Zellulose für die Gelform | ad 100,0 g |

3. Mittel nach Anspruch 1,
dadurch gekennzeichnet,
daß die Zusammensetzung im wesentlichen wie folgt ist :

| Milchsäure | 5,0 g |
| Na OH (5M) | 4,1 g |
| Glycogen | 0,1 g |
| PropylenglycolO) (85 %, Rest H$^2$ | 15,0 g |
| Konsistenzmittel wie Methylhydroxypropyläther aus Zellulose (2,5 %, Rest H$_2$O) für die Cremeform | ad 100,0 g |

**Revendications**

1. Agent destiné à renforcer les mécanismes de protection naturelle de la muqueuse vaginale en contribuant à restaurer l'environnement protecteur des lactobactéries, l'agent se présentant sous la forme d'une crème, d'un gel ou d'un ovule et contenant de l'acide lactique ainsi qu'une substance tampon, la teneur en acide lactique et en substance tampon étant telle que le pH est compris dans la plage de 3,5 à 4 et est de préférence proche de 3,8, l'agent contenant un substrat de croissance des lactobactéries et des véhicules qui sont d'un type inerte dans ce contexte et qui fournissent une consistance adaptée à la forme de l'agent, caractérisé en ce que l'acide lactique présente une proportion en poids, par rapport au substrat de croissance, comprise dans les rapports pondéraux de 20 : 1 à 500 : 1, de préférence égale à 50 : 1, le substrat de croissance étant du glycogène.

2. Agent selon la revendication 1, caractérisé en ce que sa teneur est sensiblement la suivante :

| Acide lactique | 5,0 g |
| NaOH destiné à tamponner l'acide lactique à un pH de sensiblement 3,7 à 3,8 | |
| Glycogène | 0,1 g |
| Propylèneglycol (85 %, le reste étant H$_2$O) | 15,0 g |
| Agent de consistance tel qu'éther méthylhydroxypropylique de cellulose | q.s.p. 100 g |

donnant une présentation sous forme de gel.

3. Agent selon la revendication 1, caractérisé en ce que sa teneur est sensiblement la suivante :

| | |
|---|---|
| Acide lactique | 5,0 g |
| NaOH (5M) | 4,1 g |
| Glycogène | 0,1 g |
| Propylèneglycol (85 %, le reste étant $H_2O$) | 15,0 g |
| Agent de consistance tel qu'éther méthylhydroxypropylique de cellulose (2,5 %, le reste étant $H_2O$) | q.s.p. 100 g |

donnant une présentation sous forme de crème.